# EUROPEAN PATENT APPLICATION

(11) **EP 4 790 050 A1**
(43) Date of publication of application: **12.08.2026**
(21) Application number: 24888054.4
(22) Date of filing: 07.11.2024
(51) Int. Cl.: C07D 207/48, C07C 303/44

(54) **METHOD FOR PREPARING 1-[5-(2-FLUOROPHENYL)-1-{[3-(3-METHOXYPROPOXY)PHENYL]SULFONYL}-1H-PYRROL-3-YL]-N-METHYLMETHYLAMINE AND HYDROCHLORIDE THEREOF**

(30) Priority: 09.11.2023 CN 202311506706
(71) Applicant: Jiangsu Carephar Pharmaceutical Co., Ltd, Jiangsu 210000 (CN)
(72) Inventor: QIN, Yinlin, Nanjing, Jiangsu 210000 (CN); SU, Mei, Nanjing, Jiangsu 210000 (CN); WANG, Zhiqiang, Nanjing, Jiangsu 210000 (CN)
(74) Representative: Ullrich & Naumann PartG mbB
(86) International application number: PCT/CN2024/130537
(87) International publication number: WO 2025/098443

(57) **Abstract**

The present disclosure provides a method for preparing 1-[5-(2-fluorophenyl)-1-{[3-(3-methoxypropoxy)phenyl]sulfonyl}-1*H*-pyrrol-3-yl]-*N*-methylmethylamine and hydrochloride thereof. The method comprises an optimized post-treatment in preparation of 3-(3-methoxypropoxy)benzenesulfonyl chloride (H2), which results in high purity of its sodium sulfonate salt through simple impurity removal and thus high purity and stable quality of 3-(3-methoxypropoxy)benzenesulfonyl chloride (H2) through reaction with a chlorinating agent. The method comprises preparing 5-(2-fluorophenyl)-1-{[3-(3-methoxypropoxy)phenyl]sulfonyl}-1*H-*pyrrole-3-carbaldehyde (H3) under the action of a base with addition of a small amount of catalyst, but achieving a higher reaction conversion rate. The 1-[5-(2-fluorophenyl)-1-{[3-(3-methoxypropoxy)phenyl]sulfonyl}-1*H*-pyrrol-3-yl]-N-methylmethylamine and hydrochloride thereof prepared by this method have low impurity content and high yield, making them suitable for industrial production.

## Description

The present application claims priority to an earlier application, filed with the China National Intellectual Property Administration on November 9, 2023, with the patent application number 202311506706.0, and titled "METHOD FOR PREPARING PYRROLE SULFONYL COMPOUNDS". The entire content of the earlier application is incorporated herein by reference.

### TECHNICAL FIELD

The present application pertains to the field of chemical synthesis and specifically relates to a method for preparing 1-[5-(2-fluorophenyl)-1-{[3-(3-methoxypropoxy)phenyl]sulfonyl}-1*H*-pyrrol-3-yl]-*N*-methylmethylamine and hydrochloride thereof.

### BACKGROUND OF THE INVENTION

The chemical name of Keverprazan Hydrochloride is 1-[5-(2-fluorophenyl)-1-{[3-(3-methoxypropoxy)phenyl]sulfonyl}-1*H*-pyrrol-3-yl]-*N*-methylmethanamine hydrochloride, which is a potassium ion competitive acid blocker (P-CABs). Its structure is shown in Formula I. Currently, this hydrochloride is clinically used for the prevention and treatment of gastric acid-related diseases, including, but not limited to, gastrointestinal diseases such as peptic ulcer, Zollinger-Ellison syndrome, gastritis, gastric ulcer, duodenal ulcer, ulcers caused by non-steroidal anti-inflammatory drugs, Helicobacter pylori infection, gastroesophageal reflux disease, and reflux esophagitis.

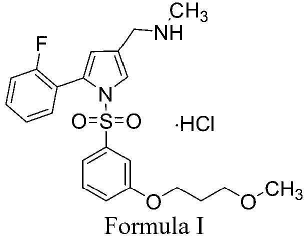

The method for preparing 1-[5-(2-fluorophenyl)-1-{[3-(3-methoxypropoxy)phenyl]sulfonyl}-1*H*-pyrrol-3-yl]-*N*-methylmethylamine disclosed in Chinese patent CN107879964B is as follows: 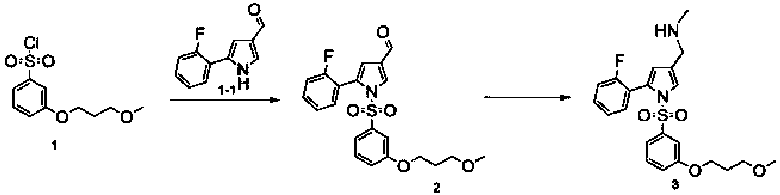 with reagents and conditions being: (a) sodium hydride, tetrahydrofuran, -30°C±5°C, yield: 66%; (b) methylamine, methanol, sodium triacetoxyborohydride, yield: 88.2%.

This method reports the synthesis of 1-[5-(2-fluorophenyl)-1-{[3-(3-methoxypropoxy)phenyl]sulfonyl}-1*H*-pyrrol-3-yl]-*N*-methylmethylamine from 3-(3-methoxypropoxy)benzenesulfonyl chloride through reaction steps including sulfonamidation and reductive amination. This scheme produces more by-products and has high energy consumption, making it unsuitable for industrial production.

The above preparation method disclosed in the prior art produces a high content of impurities, which cannot meet the pharmaceutical quality standards and the safety requirements for large-scale production. Therefore, further improvements to the preparation method are necessary.

### SUMMARY OF THE INVENTION

The present disclosure provides a method for preparing 1-[5-(2-fluorophenyl)-1-{[3-(3-methoxypropoxy)phenyl]sulfonyl}-1*H*-pyrrol-3-yl]-*N*-methylmethanamine and hydrochloride thereof. The products prepared by this method have low impurity content and high yield, making them suitable for industrial production.

The technical scheme employed by the present disclosure is as follows: 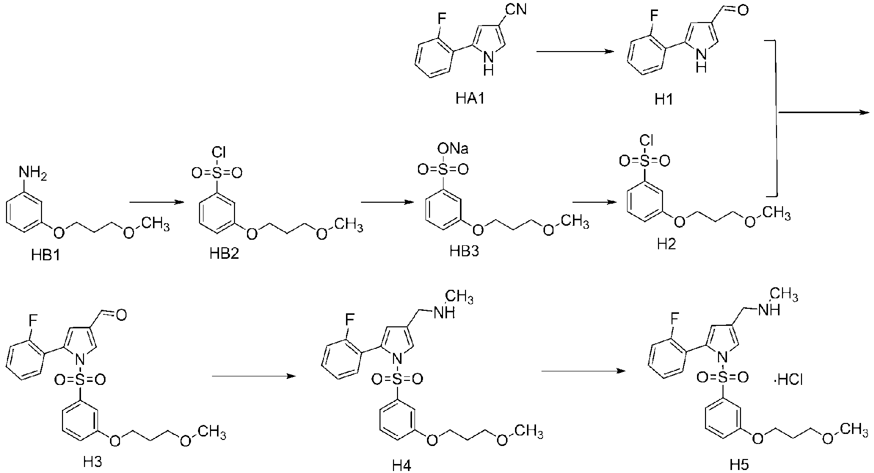
**Step 1: preparation of 5-(2-fluorophenyl)-1*H*-pyrrole-3-carbaldehyde (H1)**
   dissolving 5-(2-fluorophenyl)-1*H*-pyrrole-3-carbonitrile (HA1) in an organic solvent, performing a reduction and hydrolysis reaction under heating in the presence of Raney nickel and water with hydrogen gas introduced, and upon completion of the reaction, performing post-treatment and refining of the resultant to obtain 5-(2-fluorophenyl)-1*H*-pyrrole-3-carbaldehyde (H1) of high purity;
**Step 2: preparation of 3-(3-methoxypropoxy)benzenesulfonyl chloride (H2)**
   reacting a solution of 3-(3-methoxypropoxy)aniline (HB1) in acetic acid with sodium nitrite under the action of hydrochloric acid at a low temperature to obtain a diazotized intermediate product, performing a chlorosulfonation reaction by interacting the diazotized intermediate product with a sulfur dioxide-acetic acid solution, catalyzed by cuprous chloride at a controlled temperature for 1 h, and performing post-treatment of the resultant to obtain crude 3-(3-methoxypropoxy)benzenesulfonyl chloride (HB2);
   reacting a solution of the crude product in methanol with sodium hydroxide under heating for 40 minutes, and performing post-treatment and refining of the resultant to obtain sodium 3-(3-methoxypropoxy)benzenesulfonate (HB3);
   dissolving HB3 in an organic solvent, performing a reaction in the presence of a chlorinating reagent and a catalyst at a low temperature for 1-3 h, and performing post-treatment of the resultant to obtain 3-(3-methoxypropoxy)benzenesulfonyl chloride (H2) with high purity.
**Step 3: preparation of 5-(2-fluorophenyl)-1-{[3-(3-methoxypropoxy)phenyl]sulfonyl}-1*H*-pyrrole-3-carbaldehyde (H3)**
   dissolving 5-(2-fluorophenyl)-1*H*-pyrrole-3-carbaldehyde (H1) in an organic solvent to obtain a solution, stirring the solution for 30~40 min at controlled temperature, adding 3-(3-methoxypropoxy)benzenesulfonyl chloride (H2), performing a reaction in the presence of an organic base and a catalyst, and after completion of the reaction, performing post-treatment and refining of the resultant with an organic solvent to obtain 5-(2-fluorophenyl)-1-{[3-(3-methoxypropoxy)phenyl]sulfonyl}-1*H*-pyrrole-3-carbaldehyde (H3) with high purity;
**Step 4: 1-[5-(2-fluorophenyl)-1-{[3-(3-methoxypropoxy)phenyl]sulfonyl}-1*H*-pyrrol-3-yl]-*N*-methylmethanamine (H4)**
   subjecting a solution of 5-(2-fluorophenyl)-1-{[3-(3-methoxypropoxy)phenyl]sulfonyl}-1*H-*pyrrole-3-carbaldehyde (H3) in an organic solvent to a reaction with methylamine solution in methanol for 0.5 h and further to a reduction reaction in the presence of sodium triacetylborohydride for 1~2 h, and performing post-treatment of the resultant to obtain 1-[5-(2-fluorophenyl)-1-{[3-(3-methoxypropoxy)phenyl]sulfonyl}-1*H*-pyrrol-3-yl]-*N*-methylmethylamine (H4).
**Step 5: 1-[5-(2-fluorophenyl)-1-{[3-(3-methoxypropoxy)phenyl]sulfonyl}-1*H*-pyrrol-3-yl]-*N*-methylmethanamine hydrochloride (H5)**
   stirring a solution of 1-[5-(2-fluorophenyl)-1-{[3-(3-methoxypropoxy)phenyl]sulfonyl}-1H-pyrrol-3-yl]-*N*-methylmethanamine (H4) in an organic solvent with hydrochloric acid to yield a salt thereof, and performing post-treatment and refining of the resultant to obtain 1-[5-(2-fluorophenyl)-1-{[3-(3-methoxypropoxy)phenyl]sulfonyl}-1*H*-pyrrol-3-yl]-*N*-methylmethylamine hydrochloride (H5), i.e., Keverprazan Hydrochloride.

**The reagents, reaction conditions, and post-treatment used in the preparation steps of 1-[5-(2-fluorophenyl)-1-{[3-(3-methoxypropoxy)phenyl]sulfonyl}-1*H*-pyrrol-3-yl]-*N-*methylmethylamine hydrochloride (Keverprazan Hydrochloride) as described above are as follows:**

In step 1, the reaction solvent for the catalytic reduction of 5-(2-fluorophenyl)-1*H*-pyrrole-3-carbonitrile (HA1) is an organic weak acid such as formic acid and acetic acid, along with the addition of weak bases like pyridine, sodium phosphite, and *N*, *N*-diphenylethylenediamine to passivate Raney Ni. The combination of acetic acid and pyridine is preferred. The catalyst used is wet Raney Ni, with an amount ranging from 0.05 to 0.3 times the mass of HA1, preferably 0.16 times. Raney Ni is added after mixing with water, where the weight of water added is 1 to 10 times that of Raney Ni, preferably 5 times. The reaction hydrogen pressure ranges from atmospheric pressure (0.1 MPa) to 1.0 MPa, preferably 0.3 to 0.4 MPa. The reaction temperature is between 20 and 75°C, preferably 30 to 50°C.

The post-treatment in Step 1 includes: (1) Upon completion of the reaction, the reaction mixture is added to water for crystallization, and a solid is obtained by centrifugation; (2) The solid is dissolved in ethyl acetate, adjusted to pH 2~3 with 2 mol/L dilute hydrochloric acid, and extracted to obtain an organic phase; the organic phase is adjusted to pH 8~9 with 10% NaHCO₃, extracted again to obtain another organic phase, which is concentrated under reduced pressure to dryness to obtain a concentrate; (3) To the concentrate an organic solvent such as ethanol, tetrahydrofuran, and ethyl acetate is added for recrystallization or slurrying refining, preferably using ethyl acetate as the refining solvent; the amount of ethyl acetate is 0.5 to 2 times the weight of HA1, preferably 0.9 times; the refining condition are stirring at a temperature of 50~80°C, preferably 70~80°C, for 0.5~1 h, cooling and crystallization at a temperature of 0~10°C, stirring for 0.5~3 h, preferably 2 h, and then centrifuging and drying, so as to obtain 5-(2-fluorophenyl)-1*H*-pyrrole-3-carbaldehyde (H1).

In step 2, the diazotization reaction temperature of 3-(3-methoxypropoxy)aniline (HB1) is - 15°C~15°C, preferably -5°C~5°C; the reaction molar ratio of HB1 to sodium nitrite is 1:1~4, preferably 1:1.5; the molar ratio of HB1 to sulfur dioxide introduced is 1:1.6~7.2, preferably 1:4.8; the chlorosulfonation reaction temperature described in the preparation method is 0~40°C, preferably 20~30°C; the reaction temperature of 3-(3-methoxypropoxy)benzenesulfonyl chloride (HB2) with sodium hydroxide is 10~65°C, preferably 25~40°C; the reaction molar ratio is 1:1~3, preferably 1:2; the solvent for the reaction of 3-(3-methoxypropoxy)benzenesulfonate (HB3) with the chlorinating agent is dichloromethane, chloroform, acetonitrile, or free of solvent; the chlorinating agent is oxalyl chloride, thionyl chloride, phosphorus oxychloride, phosphorus pentachloride, triphosgene, or the like, preferably oxalyl chloride; the catalyst is *N*,*N*-dimethylformamide, *N*,*N*-dimethylaniline, pyridine, or the like, preferably *N*,*N*-dimethylformamide; the reaction molar ratio of sodium 3-(3-methoxypropoxy)benzenesulfonate to the chlorinating agent is 1:1~3, preferably 1:3; the mass ratio of sodium 3-(3-methoxypropoxy)benzenesulfonate to the catalyst is 1:1~1.2, preferably 1:1; and the reaction temperature is -30~0°C, preferably -10~0°C.

Post-treatment for HB2: Upon completion of the reaction, water is added to quench the reaction mixture, and the mixture is extracted twice with methyl tert-butyl ether to obtain an organic phase. The organic phase is washed four times with 10% sodium chloride solution, dried over anhydrous sodium sulfate, and the solvent is removed. The solvent is further removed twice with toluene to obtain a concentrate, and the concentrate is extracted four times with petroleum ether. The resultant is then filtered using a vacuum filter lined with silica gel and anhydrous sodium sulfate to obtain a filtrate. The solvent is removed from the filtrate to yield 3-(3-methoxypropoxy)benzenesulfonyl chloride (HB2).

Post-treatment for HB3: Upon completion of the reaction, the reaction mixture is vacuum filtered to obtain a filtrate. After removing the solvent from the filtrate, ethyl acetate is added. Through stirring at low temperature, centrifugation and drying, a crude product is obtained. The refining involves adding methanol in an amount of 0.5 times the mass of the crude product, controlling the temperature at 35~45°C, slowly adding ethyl acetate in an amount of 6 times the mass of the crude product while stirring, stirring for 10 minutes, cooling down by 5~10°C, stirring for 15 minutes, centrifuging, washing the filter cake, and drying, so as to obtain 3-(3-methoxypropoxy)benzenesulfonate (HB3).

Post-treatment for H2: Upon completion of the reaction, dichloromethane and water are added for extraction to obtain an organic phase, where the extraction with dichloromethane is preferably carried out three times. The organic phase is washed with water, dried over anhydrous sodium sulfate, and the solvent is removed. Then, the resultant is dissolved in ethyl acetate, washed five times with a saturated brine, and dried to remove the solvent, yielding 3-(3-methoxypropoxy)benzenesulfonyl chloride (H2).

In step 3, 5-(2-fluorophenyl)-1*H*-pyrrole-3-carbaldehyde (H1) is dissolved in an organic solvent selected from one of acetonitrile, dichloromethane, *N*,*N*-dimethylformamide, and *N-*methylpyrrolidone, preferably acetonitrile, with a solvent amount of 3.92 times that of 5-(2-fluorophenyl)-1*H*-pyrrole-3-carbaldehyde; the base is triethylamine, *N*,*N*-diisopropylethylamine, *N-*methylmorpholine, 2,6-lutidine, sodium hydroxide, cesium carbonate, tetrabutylammonium hydrogen sulfate, or the like, preferably *N*,*N*-diisopropylethylamine, with a molar ratio of 1~1.6:1 to 5-(2-fluorophenyl)-1*H*-pyrrole-3-carbaldehyde, preferably 1.4:1; the catalyst is 4-dimethylaminopyridine (DMAP), pyridine, 4-pyrrolidinopyridine (PPY), preferably DMAP, with a molar ratio of 0.1~0.3:1 to 5-(2-fluorophenyl)-1*H*-pyrrole-3-carbaldehyde, preferably 0.1:1; the reaction temperature is -5~35°C, preferably 25~35°C; the molar ratio of 5-(2-fluorophenyl)-1*H-*pyrrole-3-carbaldehyde to 3-(3-methoxypropoxy)benzenesulfonyl chloride is 1:1~1.5, preferably 1:1.2.

The post-treatment in step 3: Upon completion of the reaction, while controlling the temperature at 0~10°C, quenching the reaction mixture by adding it to drinking water 2.5 times the mass of 5-(2-fluorophenyl)-1*H*-pyrrole-3-carbaldehyde, and stirring for 15~20 minutes; controlling the temperature at 5~15°C, adjusting the pH to 3~5 with 0.5 mol/L hydrochloric acid, and stirring for 1 h; performing centrifugation, washing the filter cake with drinking water 5 times the mass of 5-(2-fluorophenyl)-1*H*-pyrrole-3-carbaldehyde, and drying by forced air at 35~45°C for 6~8 h to obtain a crude product; then adding 2.5 times the mass of the crude product of methyl tert-butyl ether, stirring at 50~55°C for 10~30 minutes, filtering the mixture while hot to obtain a filtrate, cooling the filtrate down to -5~0°C and stirring it for 1~2 h, further filtering the filtrate with suction, and drying the resultant under forced air at 35~45°C to obtain 5-(2-fluorophenyl)-1-{[3-(3-methoxypropoxy)phenyl]sulfonyl}-1*H*-pyrrole-3-carbaldehyde (H3).

In step 4, the molar ratio of 5-(2-fluorophenyl)-1-{[3-(3-methoxypropoxy)phenyl]sulfonyl}-1*H*-pyrrole-3-carbaldehyde (H3) to methylamine is 1:2~5, preferably 1:4; the molar ratio of H3 to STAB is 1:2~4, preferably 1:2.5; the organic solvent is methanol, and the amount of solvent is 3.95 times that of H3; the imine reaction temperature is 15~25°C; and the reduction reaction temperature is 10~20°C.

Post-treatment for step 4: Upon completion of the reaction, the reaction mixture is quenched by adding thereto drinking water 10 times the mass of H3. The temperature is controlled at 0~15°C. Then, 9 times the mass of H3 of ethyl acetate is added for extraction for three times, and the solvent is removed under reduced pressure. Afterwards, 17.2 times the mass of H3 of drinking water is added for dissolution. The aqueous solution is extracted four times with ethyl acetate, with the amount of ethyl acetate used being 3.2 times, 1.9 times, 1.9 times, and 1.4 times the mass of H3, respectively. The ethyl acetate phase is discarded, and the aqueous phase is adjusted to pH 9~10 with 2 mol/L sodium hydroxide solution. Dichloromethane is added for extraction three times, with the mass of dichloromethane being 5 times the mass of H3 each time. The resulting organic phase is washed three times with saturated sodium bicarbonate solution, with the mass of saturated sodium bicarbonate solution being 4.1 times the mass of H3 each time. The organic phase is dried over anhydrous sodium sulfate 2.8 times the mass of H3 for 2 h, filtered, and the solvent is removed under reduced pressure to obtain 1-[5-(2-fluorophenyl)-1-{[3-(3-methoxypropoxy)phenyl]sulfonyl}-1*H*-pyrrol-3-yl]-*N*-methylmethylamine (H4).

In step 5, the weight ratio of 1-[5-(2-fluorophenyl)-1-{[3-(3-methoxypropoxy)phenyl]sulfonyl}-1*H*-pyrrol-3-yl]-*N*-methylmethylamine (H4) to hydrochloric acid is 1:0.23; the salifying solvent is ethyl acetate, acetone, or tetrahydrofuran, preferably acetone; the solution is filtered through a 0.22um PTFE filter, and the salification temperature ranges from 0 to 30°C, with 10 to 15°C being preferred;

The post-treatment in step 5 involves suction filtration of the system, and the filter cake is rinsed with acetone 2.6 times the mass of H4. It is first dried at room temperature with forced air for 0.5 to 1 h, and then dried at 35 to 45°C with forced air for 6 to 8 h to obtain a crude product. The process for refining is as follows: adding to the crude product acetone 4 times the weight of the crude product, stirring the mixture at 0 to 5°C for 1 h, filtering the mixture, rinsing the filter cake with acetone 1 time the weight of the crude product, performing drying at room temperature with forced air for 0.5 to 1 h and at 35 to 45°C with forced air for 12 to 14 h to obtain 1-[5-(2-fluorophenyl)-1-{[3-(3-methoxypropoxy)phenyl]sulfonyl}-1*H*-pyrrol-3-yl]-*N*-methylmethylamine hydrochloride (Keverprazan Hydrochloride).

The technical effects achieved by the present disclosure are as follows:
(1) 3-(3-methoxypropoxy)benzenesulfonyl chloride (H2) with high purity is obtained through a stable process:
   The purification effect of H2 post-treatment (column chromatography) in patent CN107879964 B is poor, with longer time (over 12 h), making it difficult to scale up for industrial production. The present disclosure optimizes the post-treatment during preparation of H2. After simple impurity removal, the sodium sulfonate salt of H2 of high purity can be obtained. Impurities affecting the quality of the active pharmaceutical ingredient can be effectively removed. Then, through chloroacetylation, consistent high-purity 3-(3-methoxypropoxy)benzenesulfonyl chloride (H2) can be obtained.
(2) 5-(2-fluorophenyl)-1-{[3-(3-methoxypropoxy)phenyl]sulfonyl}-1*H*-pyrrole-3-carbaldehyde (H3) with high purity is obtained through a stable process:
   The patent CN107879964 B employs sodium hydride as the base in condensation, which poses a high safety risk during the production process. Using other bases (such as sodium tert-butoxide, or organic bases like triethylamine or *N*, *N*-diisopropylethylamine) results in a low conversion rate, increases the difficulty of compound purification, and is more expensive. In the present disclosure, a smaller amount of catalyst (such as 4-dimethylaminopyridine) is added under the action of an organic base (such as *N*, *N*-diisopropylethylamine) to achieve a higher conversion rate. The post-treatment purification process is easy to operate and yields better refining results. Compared to the previous process, which uses ethanol recrystallization that leads to impurities generated by the reaction between ethanol and intermediates, the present disclosure greatly improves the quality of intermediates and ensures the quality of the bulk drug ingredient.

The post-treatment process of 1-[5-(2-fluorophenyl)-1-{[3-(3-methoxypropoxy)phenyl]sulfonyl}-1*H*-pyrrol-3-yl]-*N*-methylmethylamine (H4) is improved, which enhances production operability.

The abbreviations for the reaction reagents mentioned in the specification are as follows:
DMF: *N*, *N*-dimethylformamide;
DIEPA: *N*, *N*-diisopropylethylamine;
DCM: dichloromethane;
DMAP: 4-dimethylaminopyridine;
2,6-Lutidine: 2,6-dimethylpyridine; and
Cs₂CO₃: cesium carbonate.

### DETAILED DESCRIPTION OF THE INVENTION

### Example 1: Preparation of 3-(3-methoxypropoxy)benzenesulfonyl chloride (H2)

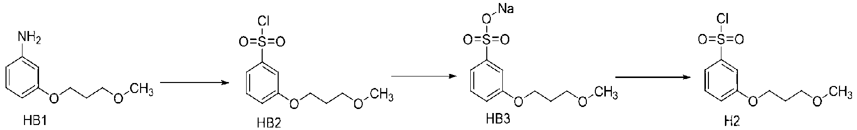

### (1) Preparation of 3-(3-methoxypropoxy)benzenesulfonyl chloride (HB2)

48.0 kg of glacial acetic acid, 12.0 kg of 3-(3-methoxypropoxy)aniline (HB1), and 20.9 kg of hydrochloric acid were added sequentially to a 300 L reaction vessel and stirred until dissolved. The system was then cooled to -5~5 °C. 7.0 kg of sodium nitrite was dissolved in 12.0 kg of drinking water and added to the reaction vessel. The mixture was kept at this temperature and stirred for 0.5 h to obtain a diazonium salt solution, which was ready for later use. 60.0 kg of glacial acetic acid was added to a 500 L reaction vessel and stirred while maintaining the temperature at 10~20 °C. 20.4 kg of sulfur dioxide was then introduced. Upon completion of the aeration, 2.4 kg of cuprous chloride was added, followed by the diazonium salt solution. After the addition was completed, the system was heated to 20~30 °C and the reaction was maintained at this temperature for 1 h. Upon completion of the reaction, 72.0 kg of drinking water was added to the system, and the mixture was extracted twice with methyl tert-butyl ether (60.0 kg * 2) to obtain an organic phase. The organic phase was washed four times with a 10% sodium chloride aqueous solution (72 kg * 4), and dried with 24.0 kg of anhydrous sodium sulfate by stirring for 0.5~1 h. The mixture was filtered, concentrated under reduced pressure to remove the solvent, and then concentrated twice under pressure by using toluene (24.0 kg * 2) to remove the solvent again, yielding a concentrate. The concentrate was extracted four times with petroleum ether at 35~45°C (48.0 kg * 4), and filtered through a vacuum filter lined with 20.0 kg of silica gel and 6.0 kg of anhydrous sodium sulfate. The filtrate was concentrated under reduced pressure to obtain 8.46 kg of a light yellow oily substance, HB2.

### (2) Preparation of sodium 3-(3-methoxypropoxy)benzenesulfonate (HB3)

16.84 kg of methanol and 8.42 kg of crude 3-(3-methoxypropoxy)benzenesulfonyl chloride (HB2) were added sequentially to a 200 L reaction vessel. The temperature was controlled at 25~40 °C, and sodium hydroxide/methanol solution (2.69 kg/8.42 kg) was slowly added. The reaction was allowed to proceed for 40 minutes. Upon completion of the reaction, the mixture was filtered, and the filter cake was washed four times with methanol (1.68 kg * 4). The filtrate was concentrated under reduced pressure, and 50.52 kg of ethyl acetate was added. The mixture was stirred at 30~40 °C for 30 minutes, then cooled to 5~10 °C and stirred for 15 minutes. After centrifugation, the filter cake was washed four times with ethyl acetate (6.74 kg * 4) and dried by forced air for 8~10 h to obtain 8.58 kg of white solid crude product. The crude product was added to a 200L reaction vessel with 4.29 kg of methanol and stirred for 10 minutes. The temperature was controlled at 35~45°C. Then, 51.48 kg of ethyl acetate was slowly added and stirred for 10 minutes. The temperature was then lowered to 5~10°C and stirred for 15 minutes. After centrifugation, the filter cake was washed twice with ethyl acetate (3.43 kg * 2) and dried by forced air at 45~55°C for 8~10 h to obtain 7.02 kg of white solid sodium 3-(3-methoxypropoxy)benzenesulfonate (HB3).

### (3) Preparation of 3-(3-methoxypropoxy)benzenesulfonyl chloride (H2)

27.3 kg of dichloromethane was added to a 100 L reaction vessel. While stirring, 6.5 kg of sodium 3-(3-methoxypropoxy)benzenesulfonate (HB3) and 6.5 kg of *N*, *N*-dimethylformamide were added sequentially. The mixture was cooled to -15 to -5 °C, and 9.1 kg of oxaloyl chloride was added dropwise. After the addition was completed, the temperature was maintained at -10 to 0 °C, and the reaction was stirred for 1 to 3 h. Upon completion of the reaction, the temperature was maintained at ≤15 °C, and 39.0 kg of drinking water was slowly added to the system. The mixture was extracted three times with dichloromethane (13.0 kg * 3) to obtain the organic phase. The organic phase was washed with 22.0 kg of drinking water, and then dried with 5.4 kg of anhydrous sodium sulfate for 1 to 2 h. The mixture was filtered, and the filter cake was rinsed with 3.0 kg of dichloromethane. The filtrate was concentrated under reduced pressure. 26.0 kg of ethyl acetate was added and stirred until completely dissolved. The solution was washed five times with saturated brine (13.0 kg * 5) to obtain the organic phase. 5.4 kg of anhydrous sodium sulfate was added and the mixture was dried for 1~2 h. The solution was filtered, and the filter cake was rinsed with 3.0 kg of ethyl acetate. The solution was concentrated under reduced pressure to obtain 4.67 kg of a pale yellow oily substance, H2. Examples and Comparative Examples of H2 preparation under different reaction conditions are shown in Tables 1 and 2 below:

**Table 1 Examples of preparing H2 under different reaction conditions**

| Example | Chlorinating reagent (equivalent to HB3) | Reaction solvent | Catalyst (mass relative to HB3) | Reaction temperature | Reaction time/h | Yield | Purity |
|---|---|---|---|---|---|---|---|
| 1.1 | oxalyl chloride 1eq | DCM | DMF1w/w | -10 to 0°C | 3 | 35.3% | 97.3% (finished product) |
| 1.2 | oxalyl chloride 2eq | DCM | DMF1w/w | -10 to 0°C | 3 | 51.8% | 98.9% (finished product) |
| 1.3 | oxalyl chloride 3eq | DCM | DMF1w/w | -10 to 0°C | 3 | 79.2% | 99.8% (finished product) |
| 1.4 | oxalyl chloride 3eq | DCM | DMF1.2w/w | -10 to 0°C | 3 | 78.2% | 97.3% (finished product) |
| 1.5 | oxalyl chloride 3eq | DCM | DMF1w/w | -20 to -10°C | 3 | 60.0% | 99.1% (finished product) |
| 1.6 | oxalyl chloride 3eq | DCM | DMF1w/w | -30 to -20°C | 3 | 50.2% | 99.6% (finished product) |
| 1.7 | oxalyl chloride 3eq | DCM | DMF1w/w | -10 to 0°C | 1 | N/A | 99.1% (reaction mixture) |
| 1.8 | oxalyl chloride 3eq | DCM | DMF1w/w | -10 to 0°C | 2 | N/A | 99.4% (reaction mixture) |
| 1.9 | oxalyl chloride 3eq | DCM | DMF1w/w | -10 to 0°C | 4 | N/A | 99.3% (reaction mixture) |
| 1.10 | oxalyl chloride 3eq | DCM | DMF1w/w | -10 to 0°C | 5 | N/A | 99.2% (reaction mixture) |
| 1.11 | oxalyl chloride 3eq | DCM | *N*,*N-*dimethylani line 1w/w | -10 to 0°C | 3 | 76.2% | 97.8% (finished product) |

**Table 2 Comparative Examples of preparing H2 under different reaction conditions**

| Comparative Example | Chlorinating reagent (equivalent relative to HB3) | Reaction solvent | Catalyst (mass relative to HB3) | Reaction temperature | Reaction time/h | Yield | Purity |
|---|---|---|---|---|---|---|---|
| 1.12 | thionyl chloride 3eq | / | / | 20~30°C | 3 | 8.1% | 94.8% (finished product) |
| 1.13 | thionyl chloride 3eq | / | / | 70~80°C | 3 | 44.5% | 96.3% (finished product) |
| 1.14 | phosphorus oxychloride 3eq | / | / | 20~30°C | 3 | 32.9% | 92.6% (finished product) |
| 1.15 | oxaloyl chloride 3eq | DCM | / | -10 to 0°C | 3 | N/A | 62.8% (reaction mixture) |

As shown in Table 1, when the molar ratio of HB3 to chlorinating agent is 1:1~3, the temperature is -10~0°C, and the mass ratio of HB3 to catalyst is 1~1.2, the purity can reach over 97%. In Example 1.3, when the temperature is -10~0°C, the molar equivalent of HB3 to chlorinating agent is 1:3, and the mass ratio of HB3 to catalyst is 1:1, both the yield and purity are the highest. From Examples 1.1~1.3, it can be seen that the equivalent of chlorinating agent affects the degree of reaction. When the molar ratio of HB3 to chlorinating agent is 1:1, there is more residual raw material, and the yield of the finished product is lower; when the molar ratio of HB3 to chlorinating agent is 1:3, both the yield and purity are the highest. From Examples 1.5~1.6, it can be seen that when the reaction temperature is lower, the yield will decrease. In Example 1.11, *N*, *N-*dimethylaniline was used as the catalyst, and the yield was not as high as that when *N*, *N-*dimethylformamide was used as the catalyst.

As shown in Table 2, when other chlorination reagents are added at a molar ratio of HB3 to chlorination reagent of 1:3, the required temperature is higher, energy consumption is greater, and both the reaction yield and purity are lower. When no catalyst is added, the purity of H2 in the reaction mixture of Comparative Example 1.14 is significantly reduced.

### Example 2: Preparation of 5-(2-fluorophenyl)-1H-pyrrole-3-carbaldehyde (H1)

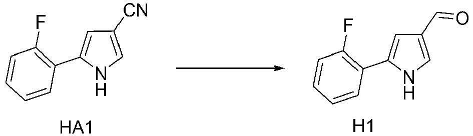

10.0 kg of 5-(2-fluorophenyl)-1*H*-pyrrole-3-carboxynitrile (HA1) was added to 10.0 kg of glacial acetic acid and 20.0 kg of pyridine, and stirred at room temperature until dissolved. 1.6 kg of Raney nickel was added to 8.0 kg of drinking water, and the mixture was successively pumped into a 300 L nitrogen-purged hydrogenation reactor. Then, hydrogen gas was introduced to replace nitrogen, and the hydrogen pressure was controlled at 0.3~0.4 MPa. The temperature was controlled at 30~50 °C, and the reaction was stirred until the hydrogen pressure remained unchanged. Upon completion of the reaction, the hydrogenation reactor was purged with nitrogen, the system was filtered, and the filtrate was added to 201.0 kg of drinking water. The temperature was controlled at 10~20 °C, and the mixture was stirred for 1 h, then filtered. The filter cake was washed with 21.0 kg of drinking water. The filter cake was dissolved in 216 kg of ethyl acetate, and the pH was adjusted to 2~3 with 2 mol/L HCl solution. The mixture was separated to obtain the organic phase. Then, 10% sodium bicarbonate aqueous solution was added to adjust the pH to 8~9. The mixture was separated to obtain the organic phase. The organic phase was concentrated under reduced pressure to dryness, and 9.0 kg of ethyl acetate was added. The temperature of the mixture was raised to 70~80°C and stirred for 0.5~1 h, and then the temperature was lowered to 0~10°C and stirred for 2 h. The mixture was filtered, and the filter cake was dried in a forced-air drying oven at 45~50°C to constant weight to obtain 5.26 kg of light brown solid H1.

### Example 3: Preparation of 5-(2-fluorophenyl)-1-{[3-(3-methoxypropoxy)phenyl]sulfonyl}-1H-pyrrole-3-carbaldehyde (H3)

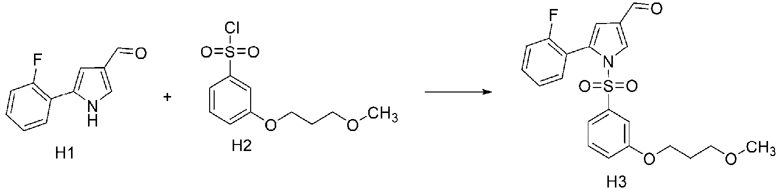

10.93 kg of acetonitrile was added to a 100 L reaction vessel, and the mixture was stirred and kept at a temperature of 25~30 °C. Then, 2.79 kg of 5-(2-fluorophenyl)-1*H*-pyrrole-3-carboxaldehyde (H1), 2.65 kg of *N*,*N*-diisopropylethylamine, and 0.19 kg of 4-dimethylaminopyridine were added sequentially, and stirred for 30~40 minutes. Then, 4.42 kg of 3-(3-methoxypropoxy)benzenesulfonyl chloride (H2) was added dropwise. After the addition was completed, the mixture was kept at a constant temperature and stirred for 13~15 h. Upon completion of the reaction, the temperature was maintained at 0~10 °C, and the reaction solution was slowly added to 6.98 kg of drinking water with stirring for 15~20 minutes to quench the reaction. Then, the temperature was maintained at 5~15 °C, and 0.5 mol/L HCl solution was added dropwise to adjust the pH to 3~5, and the mixture was stirred for 1 h. Centrifugation was performed, and the filter cake was rinsed with 13.95 kg of drinking water and dried by forced air at 35~45°C for 6~8 h to obtain 4.86 kg of crude product. 12.13 kg of methyl tert-butyl ether was added to the crude product, and the mixture was stirred at 50~55°C for 10~30 minutes. The mixture was filtered while hot, and the filtrate was transferred to another 100 L reaction vessel. After cooling to -5~0°C by stirring, the mixture was kept at this temperature and stirred for 1~2 h. The mixture was then filtered, and dried by forced air at 35~45°C for 3~5 h to obtain 4.62 kg of an off-white solid. Examples and Comparative Examples of H3 preparation under different reaction conditions are shown in Tables 3 and 4 below:

**Table 3 Examples of preparing H3 under different reaction conditions**

| Example | Base (equivalent relative to H1) | Catalyst (equivalent relative to H1) | Reaction temperature | Reaction solvent | H2 (equivalent relative to H1) | Yield | Purity | Properties |
|---|---|---|---|---|---|---|---|---|
| 3 | 1.4eq DIPEA | 0.1eq DMAP | 25 to 30°C | Acetonitrile | 1.2eq | 75.0% | 99.8% | off-white solid |
| 3.1 | 1.4eq DIPEA | 0.1eq DMAP | 25 to 30°C | Acetonitrile | 1.2eq | 88.3% | 99.1% | Brown to reddis h-brow n solids ; the pigme nt will be carrie d over to the next inter media te. |
| 3.2 | 1.4eq DIPEA | 0.2eq DMAP | 25 to 30°C | Acetonitrile | 1.2eq | 85.8% | 99.3% | |
| 3.3 | 1.2eq DIPEA | 0.2eq DMAP | 25 to 30°C | Acetonitrile | 1.2eq | 66.2% | 97.6% | |
| 3.4 | 1.6eq DIPEA | 0.2eq DMAP | 25 to 30°C | Acetonitrile | 1.2eq | 77.4% | 99.1% | |
| 3.5 | 1.4eq DIPEA | 0.3eq DMAP | 25 to 30°C | Acetonitrile | 1.2eq | 79.6% | 98.9% | |
| 3.6 | 1.4eq DIPEA | 0.1eq DMAP | 25 to 30°C | Acetonitrile | 1.1eq | 73.9% | 96.9% | |
| 3.7 | 1.4eq DIPEA | 0.1eq DMAP | 25 to 30°C | Acetonitrile | 1.0eq | 68.2% | 95.7% | |
| 3.8 | 1.4eq DIPEA | 0.1eq DMAP | 25 to 30°C | Acetonitrile | 1.5eq | 83.6% | 98.5% | |
| 3.9 | 1.4eq DIPEA | 0.1eq DMAP | -5 to 0°C | Acetonitrile | 1.2eq | 63.8% | 96.2% | |
| 3.10 | 1.4eq DIPEA | 0.1eq DMAP | 10 to 15°C | Acetonitrile | 1.2eq | 74.9% | 98.6% | |
| 3.11 | 1.4eq DIPEA | 0.1eq Pyridine | 25 to 30°C | Acetonitrile | 1.2eq | 68.6% | 99.0% | |
| 3.12 | 1.4eq DIPEA | 0.1eq 4-Pyrrolidinyl pyridine | 25 to 30°C | Acetonitrile | 1.2eq | 63.7.% | 98.8% | |
| 3.13 | 1.0eq 2,6-Lutidine | 0.1eq DMAP | 25 to 30°C | Acetonitrile | 1.2eq | 63.9% | 96.6% | |
| 3.14 | 1.4eq 2,6-Lutidine | 0.1eq DMAP | 25 to 30°C | Acetonitrile | 1.2eq | 71.3% | 98.1% | |
| 3.15 | 1.4eq Cs₂CO₃ | 0.1eq DMAP | 25 to 30°C | Acetonitrile | 1.2eq | 75.8% | 97.8% | |
| 3.16 | 1.4eq DIPEA | 0.1eq DMAP | 25 to 30°C | *N*,*N-*dimethylfor mamide | 1.2eq | 80.3% | 98.7% | |
| 3.17 | 1.4eq DIPEA | 0.1eq DMAP | 25 to 30°C | *N-*methylpyrrol idone | 1.2eq | 81.4% | 97.4% | |

**Table 4: Comparative Examples of preparing H3 under different reaction conditions**

| Comparative Example | Base (equivalent relative to H1) | Catalyst (equivalent relative to H1) | Reaction temperature | Reaction solvent | H2 (equivalent relative to H1) | Yield | Purity | Properties |
|---|---|---|---|---|---|---|---|---|
| 3.18 | 1.4eq DIPEA | 0.2eq DMAP | 25 to 30°C | Methanol | 1.2eq | N/A (No reaction) | N/A | N/A |
| 3.19 | 1.4eq DIPEA | 0.2eq DMAP | 25 to 30°C | Tetrahyd rofuran | 1.2eq | N/A (Large amount of raw materials remaining) | N/A | N/A |
| 3.20 | 1.4eq DIPEA | 0.2eq DMAP | 25 to 30°C | Ethyl acetate | 1.2eq | N/A (Large amount of raw materials remaining) | N/A | N/A |

In Table 3, Example 3 presents data obtained through recrystallization and refining using methyl tert-butyl ether; and Examples 3.1~3.17 and Comparative Examples 3.18~3.20 in Table 4 present data obtained without recrystallization and refining using methyl tert-butyl ether.

In Table 3, when the molar ratio of H1 : H2 : base : catalyst is 1:1~1.5:1~1.6:0.1~0.3, the yield is above 63% and the purity is above 95%. Although the yield decreases after recrystallization and refining with methyl tert-butyl ether in Example 3, the pigment is removed and the purity reaches 99.8%. For 3.1, when the molar ratio of H1 : base : catalyst : H2 is 1:1.4:0.1:1.2 and the temperature is 25~30°C, the reaction yield is relatively high. In Examples 3.3~3.4, too much or too little base equivalent will affect the yield, resulting in a lower yield. According to the data in Table 3, when the catalyst equivalent is 0.1~0.3, the yield is not significantly affected, and the yield is highest at 0.1. At lower temperatures, both the reaction yield and purity are affected to some extent. At the same time, an excessive amount of H2 relative to H1 will increase the degree of reaction, and when the H2 equivalent is 1.0, there is some residual raw material, affecting the yield and purity. When the H2 equivalent is too high, it will increase the amount of by-products, resulting in a lower yield. As for use of different bases and solvents, the data in the table shows that when *N*, *N-*diisopropylethylamine is used as the base and acetonitrile as the solvent, the yield and purity are higher.

In Table 4, when the reaction solvents are methanol, tetrahydrofuran, and ethyl acetate, a large amount of raw materials are remained and unreacted.

### Example 4: Preparation of 1-[5-(2-fluorophenyl)-1-{[3-(3-methoxypropoxy)phenyl]sulfonyl}-1H-pyrrol-3-yl]-N-methylmethylamine (H4)

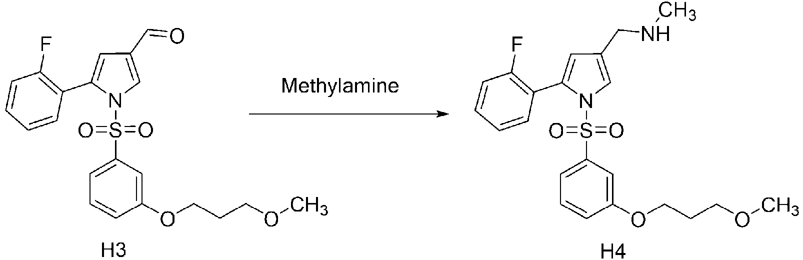

17.34 kg of methanol, 3.95 kg of methylamine (in methanol), and 4.39 kg of 5-(2-fluorophenyl)-1-{[3-(3-methoxypropoxy)phenyl]sulfonyl}-1*H*-pyrrole-3-carboxaldehyde (H3) were added sequentially to a 100 L reaction vessel. The temperature was controlled at 15~25 °C, and the reaction was stirred for 0.5 h. Then, 5.53 kg of sodium triacetoxyborohydride was slowly added while maintaining the temperature at 10~20 °C, and the reaction was carried out for 1~2 h. Upon completion of the reaction, 43.9 kg of drinking water was added to a 200 L reaction vessel. The reaction mixture was stirred and the temperature controlled at 0~15 °C. The reaction solution was slowly added to the water, and the mixture was stirred to quench the reaction for 15~20 minutes. The mixture was extracted three times with ethyl acetate (39.51 kg * 3), organic layers were combined, and then concentrated under reduced pressure. Upon completion, 75.5 kg of drinking water was added to the concentrate and stirred until dissolved completely. Ethyl acetate was then added to the concentrate in a 200 L reactor and washed four times with stirring (14.04 kg, 8.34 kg, 8.34 kg, and 6.15 kg) to obtain the aqueous phase. The temperature was controlled at 10~20 °C, and 2 mol/L sodium hydroxide solution was added dropwise to adjust the pH to 9~10. Then, dichloromethane (21.95 kg * 3) was added for three extractions, and organic phases were combined. The combined organic phase was then washed three times with saturated sodium bicarbonate aqueous solution (18.0 kg * 3), and dried with 12.29 kg of anhydrous sodium sulfate for 2 h with stirring. The mixture was vacuum filtered, and the filtrate was concentrated under reduced pressure to obtain 4.2 kg of an oily substance, which was used directly in the next step.

### Example 5: Preparation of 1-[5-(2-fluorophenyl)-1-{[3-(3-methoxypropoxy)phenyl]sulfonyl}-1H-pyrrol-3-yl]-N-methylmethylamine hydrochloride (H5)

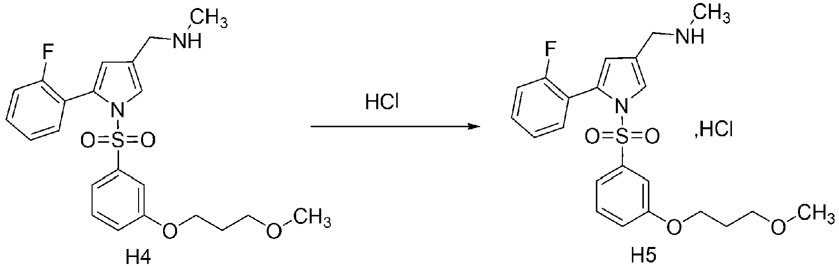

The oily substance of 1-[5-(2-fluorophenyl)-1-{[3-(3-methoxypropoxy)phenyl]sulfonyl}-1H-pyrrolo-3-yl]-*N*-methylmethylamine (H4) obtained from the previous step was dissolved in 33.18 kg of acetone and stirred until dissolved completely. The solution was subjected to compression filtration through a precision filter (using a dedicated PTFE filter element, 0.22 µm). The filtrate was added to a 100 L reaction vessel, and the temperature was maintained at 10~15 °C. 0.97 kg of hydrochloric acid was added, and the mixture was stirred for 2 h. The mixture was then subjected to suction filtration, and the filter cake was washed with 10.92 kg of acetone. The filter cake was first dried at room temperature with forced air for 0.5~1 h, then dried at 35~45 °C for 6~8 h to obtain 3.16 kg of a white solid crude product.

In a 100 L reaction vessel, 12.52 kg of acetone and 3.13 kg of the crude product were added, and the temperature was maintained at 0~5 °C with stirring for 1 h. The mixture was then subjected to suction filtration, and the filter cake was rinsed with 3.13 kg of acetone. The filter cake is first dried in a hot air circulating oven with forced air for 0.5 to 1 h, then heated and dried at 35 to 45°C for 12 to 14 h. After drying, the product is weighed and passed through a 40-mesh sieve to obtain 1-[5-(2-fluorophenyl)-1-{[3-(3-methoxypropoxy)phenyl]sulfonyl}-1*H*-pyrrole-3-yl]-*N-*methylmethylamine hydrochloride (Keprashen Hydrochloride).

The above has provided a detailed description of some components of this patent. However, it is evident to those skilled in the art that such detailed description is merely intended to illustrate exemplary embodiments and should not be construed as limiting the scope of this patent. Therefore, the substantive scope of the present disclosure is defined by the appended claims and their equivalents.

## Claims

1. A method for preparing 1-[5-(2-fluorophenyl)-1-{[3-(3-methoxypropoxy)phenyl]sulfonyl}-1*H-*pyrrol-3-yl]-*N*-methylmethanamine and hydrochloride thereof, 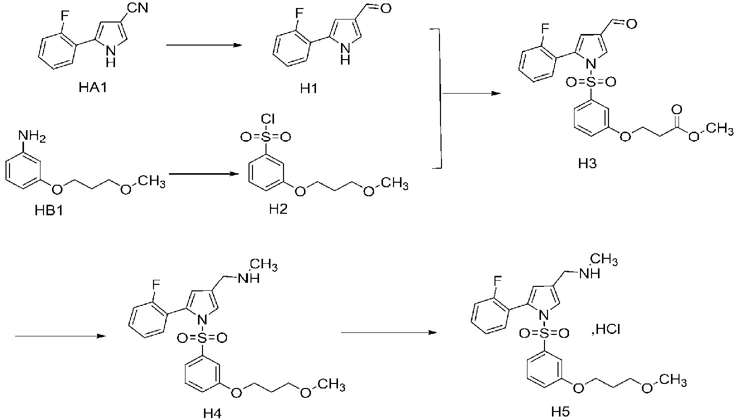 wherein the method comprises:
**Step 1: preparation of 5-(2-fluorophenyl)-1*H*-pyrrole-3-carbaldehyde (H1):**
dissolving 5-(2-fluorophenyl)-1*H*-pyrrole-3-carbonitrile (HA1) in an organic solvent, performing a reaction in the presence of a catalyst and water with hydrogen gas introduced, and after completion of the reaction, performing post-treatment and refining of the resultant to obtain 5-(2-fluorophenyl)-1*H*-pyrrole-3-carbaldehyde (H1), wherein the catalyst is Raney nickel;
**Step 2: preparation of 3-(3-methoxypropoxy)benzenesulfonyl chloride (H2):**
reacting 3-(3-methoxypropoxy)aniline (HB1) with hydrochloric acid and sodium nitrite to yield a diazotized intermediate product, which is further reacted with cuprous chloride and a solution of sulfur dioxide in acetic acid to yield crude 3-(3-methoxypropoxy)benzenesulfonyl chloride; reacting the crude 3-(3-methoxypropoxy)benzenesulfonyl chloride with a base to yield sodium 3-(3-methoxypropoxy)benzenesulfonate, which is post-treated by adding a chlorinating reagent and a catalyst to obtain 3-(3-methoxypropoxy)benzenesulfonyl chloride (H2);
**Step 3: preparation of 5-(2-fluorophenyl)-1-{[3-(3-methoxypropoxy)phenyl]sulfonyl}-1*H-*pyrrole-3-carbaldehyde (H3):**
reacting a solution of 5-(2-fluorophenyl)-1*H*-pyrrole-3-carbaldehyde (H1) in an organic solvent with 3-(3-methoxypropoxy)benzenesulfonyl chloride (H2) in the presence of a base and a catalyst, and after completion of the reaction, performing post-treatment of the resultant to obtain 5-(2-fluorophenyl)-1-{[3-(3-methoxypropoxy)phenyl]sulfonyl}-1*H*-pyrrole-3-carbaldehyde (H3), wherein the catalyst is one selected from the group consisting of 4-dimethylaminopyridine, pyridine, and 4-pyrrolidinopyridine;
**Step 4: 1-[5-(2-fluorophenyl)-1-{[3-(3-methoxypropoxy)phenyl]sulfonyl}-1*H*-pyrrol-3-yl]-***N*-methyl**methanamine (H4):**
reacting a solution of 5-(2-fluorophenyl)-1-{[3-(3-methoxypropoxy)phenyl]sulfonyl}-1*H-*pyrrole-3-carbaldehyde (H3) in an organic solvent with methylamine solution in methanol in the presence of sodium triacetylborohydride, and performing post-treatment of the resultant to obtain 1-[5-(2-fluorophenyl)-1-{[3-(3-methoxypropoxy)phenyl]sulfonyl}-1*H*-pyrrol-3-yl]-*N-*methylmethylamine (H4); and
**Step 5: 1-[5-(2-fluorophenyl)-1-{[3-(3-methoxypropoxy)phenyl]sulfonyl}-1*H*-pyrrol-3-yl]-***N*-methyl**methanamine hydrochloride (H5):**
reacting a solution of 1-[5-(2-fluorophenyl)-1-{[3-(3-methoxypropoxy)phenyl]sulfonyl}-1*H-*pyrrol-3-yl]-*N*-methylmethanamine (H4) in an organic solvent with hydrochloric acid, and performing post-treatment of the resultant to obtain 1-[5-(2-fluorophenyl)-1-{[3-(3-methoxypropoxy)phenyl]sulfonyl}-1*H*-pyrrol-3-yl]-*N*-methylmethylamine hydrochloride (H5).

2. The method according to claim 1, wherein the base in Step 3 is *N*, *N*-diisopropylethylamine, 2, 6-lutidine, or cesium carbonate.

3. The method according to claim 1, wherein the molar ratio of 5-(2-fluorophenyl)-1*H*-pyrrol-3-carbaldehyde (H1), 3-(3-methoxypropoxy)benzenesulfonyl chloride (H2), base, and catalyst in Step 3 is 1:1~1.5:1~1.6:0.1~0.3.

4. The method according to claim 1, wherein the temperature for the reaction in Step 3 is -5 to 35°C.

5. The method according to claim 4, wherein the temperature for the reaction in Step 3 is 25 to 35°C.

6. The method according to claim 1, wherein the organic solvent in Step 3 is acetonitrile, *N*, *N-*dimethylformamide, or *N*-methylpyrrolidone.

7. The method according to claim 1, wherein the post-treatment in Step 3 comprises, by the end of the reaction, quenching the reaction by adding the resultant reaction mixture into water; adjusting the pH value to 3~5 with hydrochloric acid; performing centrifugation and drying to obtain a crude product, recrystallizing the crude product with addition of methyl tert-butyl ether; thermal-filtrating the recrystallized product with removal of the filter residue; and performing centrifugation on cooled filtrate and drying to obtain 5-(2-fluorophenyl)-1-{[3-(3-methoxypropoxy)phenyl]sulfonyl}-1*H-*pyrrole-3-carbaldehyde (H3).

8. The method according to claim 1, wherein, in Step 2, the base is sodium hydroxide, the catalyst is *N*,*N*-dimethylformamide or *N*,*N*-dimethylaniline, the chlorinating agent is oxalyl chloride, thionyl chloride, phosphorus oxychloride, phosphorus pentachloride or triphosgene, and the temperature for the reaction is -30 to 0°C.

9. The method according to claim 8, wherein, in Step 2, the chlorinating agent is oxalyl chloride, and the temperature for the reaction is -10 to 0°C.

10. The method according to claim 1, wherein, in Step 2, the reaction molar ratio of sodium 3-(3-methoxypropoxy)benzenesulfonate to the chlorinating agent is 1:1~3, and the mass ratio of sodium 3-(3-methoxypropoxy)benzenesulfonate to the catalyst is 1:1~1.2; and the post-treatment comprises, by the end of the reaction, adding dichloromethane and water for extraction to yield an organic phase, washing the organic phase with water and dried it over anhydrous sodium sulfate, removing the solvent therein to obtain a crude product, and after redissolving the crude product in ethyl acetate and washing it with saturated brine, and drying it to remove the solvent to obtain 3-(3-methoxypropoxy)benzenesulfonyl chloride (H2).
